Europäisches Patentamt

European Patent Office

Office européen des brevets

(19)

(11) Veröffentlichungsnummer: **0 287 647**
**B1**

(12) **EUROPÄISCHE PATENTSCHRIFT**

(45) Veröffentlichungstag der Patentschrift: **16.01.91**

(51) Int. Cl.⁵: **A 23 L 2/40, A 61 K 9/46**

(21) Anmeldenummer: **87907312.0**

(22) Anmeldetag: **21.10.87**

(86) Internationale Anmeldenummer:
**PCT/EP87/00618**

(87) Internationale Veröffentlichungsnummer:
**WO 88/02993 05.05.88 Gazette 88/10**

(54) **VERFAHREN ZUM HERSTELLEN EINES BRAUSEGRANULATES, DANACH HERGESTELLTES BRAUSEGRANULAT SOWIE DESSEN VERWENDUNG.**

(30) Priorität: **22.10.86 DE 3635864**

(43) Veröffentlichungstag der Anmeldung:
**26.10.88 Patentblatt 88/43**

(45) Bekanntmachung des Hinweises auf die
Patenterteilung:
**16.01.91 Patentblatt 91/03**

(84) Benannte Vertragsstaaten:
**AT BE CH DE FR GB IT LI LU NL SE**

(56) Entgegenhaltungen:
**DE-A-3 149 517**
**FR-A-2 552 308**
**US-A-3 359 119**
**US-A-4 602 039**

(73) Patentinhaber: **Gergely, Gerhard, Dr.**
**Gartengasse 8**
**A-1050 Wien (AT)**

(72) Erfinder: **GERGELY, Gerhard**
**Gartengasse 8**
**A-1050 Wien (AT)**
Erfinder: **GERGELY, Irmgard**
**Gartengasse 8**
**A-1050 Wien (AT)**
Erfinder: **GERGELY, Thomas**
**Gartengasse 8**
**A-1050 Wien (AT)**
Erfinder: **WOLF, Ruediger**
**Elisabethstrasse 2**
**A-2380 Perchtoldsdorf (AT)**

(74) Vertreter: **Goddar, Heinz J., Dr. et al**
**FORRESTER & BOEHMERT Widenmayerstrasse 4/I**
**D-8000 München 22 (DE)**

Courier Press, Leamington Spa, England.

## Beschreibung

Die Erfindung betrifft ein Verfahren zum Herstellen eines Brausegranulates nach dem Oberbegriff des Patentanspruches 1, ein danach hergestelltes Brausegranulat sowie dessen Verwendung.

Der wünschenwerte Zusatz der vitaminähnlichen Verbindung L-Carnitin, welches eine bedeutende Rolle im menschlichen Metabolismus spielt, zu Brausegranulaten ist aufgrund der starken Hygroskopizität dieser Substanz bisher nicht praktibel gewesen. L-Carnitin erfüllt eine wesentliche Funktion für die Verwertung von Fettsäuren und für den Transport von Stoffwechselenergie.

Der Betain-Charakter, d.h. die Ausbildung eines inneren Salzes zwischen dem positiv geladenen Stickstoff und der negative geladenen COO-Gruppe im Carnitin ist der Grund für dessen Reaktionsfähigkeit mit Säuren sowie seine starke Hygroskopizität. Die kommerziell erhältlichen pharmazeutischen Carnitin-Zubereitungen vermischen daher Carnitin mit inerten Stoffen, um die Reaktionsmöglichkeiten zu verhindern. Auch für die Tablettierung sind hauptsächlich inerte Stoffe vorgeschlagen worden. Hohe Dosierungen, wie z.B. die orale Verabreichung von etwa 1 g Carnitin in Form einer Tablette oder einer Kapsel ist aber aufgrund des Volumens kaum mehr möglich. Auch eine Verdünnung mit Zucker, Kohlehydraten und anderen inerten Stoffen zum Zwecke der Herstellung einer Grundlage für die Bereitung einer Trinklösung resultiert in zu hohen Gesamtvolumina und Gewichten. Diese Grundlage ist geschmacklich nicht akzeptabel und nicht rationell.

Es wird aber angestrebt, diese höheren Dosierungen vor der Einnahme in Wasser aufzulösen.

Diese moderne Darreichungsform für höhere Dosierungen von pharmazeutischen Wirkstoffen sollte sich aber schnell und in Form eines wohlschmeckenden Getränkes auflösen. Derartige Zubereitungen benötigen aber bekanntlich neben einer raschen Auflösung auch eine geschmackliche Akzeptanz, bedingt durch organische Säuren vom Typ Weinsäure, Zitronensäure, Äpfelsäure, etc. sowie Carbonate oder Bicarbonate vom Typ Natriumcarbonat, Calciumcarbonat, etc.

Kommt nun L-Carnitin mit organischen Säuren in Kontakt, bilden sich, wie in der EP—OS—0 150 688 beschrieben, saure Salze, die eine weitaus geringere Hygroskopizität als L-Carnitin selbst aufweisen sollen. Diese sauren Salze müßten aber jetzt auf dieser Stufe isoliert werden, da sie ansonsten weiter mit der organischen Säure reagieren und am Ende eine glasige, noch wesentlich hygroskopischere Struktur ergeben als das L-Carnitin selbst.

Der Erfindung liegt daher die Aufgabe zugrunde, trotz der Säurereaktivität des L-Carnitins stabile Darreichungsformen mit organischen Säuren herzustellen, die temperaturstabil und wenig hygroskopisch sind, sich aber schnell in Wasser auflösen.

Erfindungsgemäß wird diese Aufgabe in Weiterbildung des gattungsgemäßen Verfahrens, wie es in DE—A—3627475 beschrieben ist, durch die im Kennzeichen des Patentanspruches 1 aufgeführten Merkmale gelöst.

Besonders bevorzugte Ausführungsformen des erfindungsgemäßen Verfahrens sind Gegenstand der Patentansprüche 2 bis 4. Das erfindungsgemäße Brausegranulat ist Gegenstand des Patentanspruches 5, während dessen Verwendung Gegenstand der Patentansprüche 6 und 7.

Durch die erfindungsgemäße Passivierung der Oberfläche der organischen Säurekristalle kann deren Einfluß auf das L-Carnitin verhindert werden, wobei die zusätzliche Trocknungsfunktion der äußeren Schicht der mit einem überzug versehenen Säurekristalle der Hygroskopizität von L-Carnitin entgegenwirkt. Auf diese Weise erhält man schnell lösliche Mischungen oder Tabletten, bei denen der Einfluß der organischen Säure auf L-Carnitin ausgeschaltet ist und die zusätzlich temperaturstabil und wenig hygroskopisch sind.

Besonders vorteilhaft ist es dabei, auf eine mit Puffergranulierlösung versehene Kristalloberfläche von Zitronensäure wasserfreies Natriumcarbonat in feinst pulverisierter Form aufzubringen. Verwendet man beispielsweise eine Pufferlösung von Mono-Calciumcitrat in konzentrierter Form, dann ist es möglich, größere Mengen an wasserfreiem Natriumcarbonat an der Oberfläche der Zitronensäurekristalle zu binden, ohne daß das Natriumcarbonat dabei Kristallwasser aufnehmen kann. Zum einen erfordert der Einbau von Kristallwasser im Natriumcarbonat längere Zeit, zum anderen ist der Einbau von Kristallwasser durch die konzentrierte Lösung von Mono-Calciumcitrat behindert.

Man kann also durch eine Puffergranulierung der Oberflächen von Zitronensäurekristallen und anschließendem Aufbringen von wasserfreiem Natriumcarbonat Partikel erzeugen, die auch beim Pressen zu Tabletten äußerlich alkalisch reagieren, sich aber beim Kontakt mit Wasser stürmisch auflösen.

Gemische eines derartigen Granulats mit L-Carnitin sind nun überraschenderweise kaum mehr hygroskopisch und lösen sich sowohl in Form eines konzentrierten Granulates als auch in form von Brausetabletten schnell und klar in Wasser auf. Durch das erfindungsgemäße Verfahren ist es nun erstmals möglich geworden, Brausegranulate oder -tabletten herzustellen, die sich mit einem resultierenden End-pH zwischen 4 und 5 auflösen, die färbbar sind, mit Aromen und Süßstoffen versehen werden können und es ermöglichen, etwa 1 g L-Carnitin in einer Gesamtmenge von etwa 4 bis 5 g eines Granulates oder einer Tablette marktgerecht und rationell unterzubringen. Auf diese Weise ist es möglich, ohne den Umweg über saure Salze oder dergleichen direkt das wesentlich billigere L-Carnitin als solches zu verwenden und es durch das erfindungsgemäße Verfahren in die ebenfalls billigeren und rationell zu verwendenden Instant-Komplexe einzubauen.

Die in der vorstehenden Beschreibung und in den Ansprüchen offenbarten Merkmale der Erfindung können sowohl einzeln als auch in beliebiger Kombination für die Verwirklichung der Erfindung in ihren verschiedenen Ausführungsformen wesentlich sein.

**Patentansprüche**

1. Verfahren zum Herstellen eines Brausegranulates mit einem Gehalt an wenigstens einer festen, kristallinen, eßbaren organischen Säure, insbesondere Zitronensäure, und mindestens einem bei Reaktion mit der organischen Säure in wässriger Lösung $CO_2$-abspaltenden Alkali- oder Erdalkalimetallcarbonat, bei dem, ggf. in einer Vakuummischmaschine, zur Ausbildung einer ersten über eine durch Reaktion von Säure und Carbonat gebildete Bindeschicht an der Oberflächenschicht des jeweiligen Säurekristalls hauftenden Überzugsschicht mit einer ersten pulverisierten Überzugsmasse mit einem Gehalt an mindestens einem Alkali- oder Erdalkalimetallcarbonat ein Teil der gesamten Säuremenge und ein Teil des/der Alkali- und/oder Erdalkalimetallcarbonate(s) der ersten Überzugsmasse in einem Lösungsmittel für die Säure, wie Wasser, Alkohol oder einer Mischung hieraus, gelöst und miteinander zur Reaktion gebracht werden, woraufhin die so hergestellte Vorreaktionslösung auf die Säurekristalle aufgebracht wird und nach Bildung der ersten Überzugsschicht in mindestens einem weiteren Anreaktionsschritt mindestens eine weitere feste pulverisierte Überzugsmasse zugesetzt und an der im vorausgehenden Anreaktionsschritt gebildeten, durch das bei der Anreaktion abgespaltene Kristallwasser feuchten vorangehenden Überzugsschicht zur Ausbildung mindestens einer weiteren Überzugsschicht unter intensivem Rühren anreagiert wird, woraufhin schließlich in abbrechender Überzugsbildung eine Endtrocknung erfolgt, dadurch gekennzeichnet, daß auf die mit der Vorreaktionslösung benetzten Säurekristalle ein Salz aufgebracht wird, das in der Lage ist, in dem Gesamtkomplex Wasser irreversibel zu binden; und daß zu den mit dem Überzug versehenen Säurekristallen L-Carnitin zugesetzt wird.

2. Verfahren nach Anspruch 1, dadurch gekennzeichnet, daß auf die mit der Vorreaktionslösung benetzten Säurekristalle wasserfreies Natriumcarbonat in feinstpulverisierter Form aufgebracht wird.

3. Verfahren nach Anspruch 1 oder 2, dadurch gekennzeichnet, daß das L-Carnitin in einer Menge zugesetzt wird, daß das Brausegranulat 15 bis 30 Gew.-% L-Carnitin enthält.

4. Verfahren nach Anspruch 3, dadurch gekennzeichnet, daß das L-Carnitin in einer Menge zugesetzt wird, daß das Brausegranulat 20 bis 25 Gew.-% L-Carnitin enthält.

5. Brausegranulat, hergestellt nach einem der vorangehenden Ansprüche.

6. Verwendung des Brausegranulates nach Anspruch 5, als Instant-Granulat zum Herstellen von Brausegetränken oder dergleichen.

7. Verwendung des Brausegranulates nach Anspruch 5 zum Herstellen von Brausetabletten.

**Revendications**

1. Procédé de préparation d'un granulé effervescent à teneur en au moins un acide organique comestible, cristallin, solide, en particulier de l'acide citrique et en au moins un carbonate de métal alcalin ou alcalino-terreux dégageant $CO_2$, en solution aqueuse, par réaction avec l'acide organique, dans lequel, pour former une première couche de revêtement adhérant à la couche de surface de chaque cristal d'acide, par l'intermédiaire d'une couche de liaison formée par réaction d'acide et de carbonate, couche de revêtement ayant une première masse de revêtement pulvérisée à teneur en au moins un carbonate de métal alcalin ou alcalino-terreux, on dissout, éventuellement dans une machine mélangeuse sous vide, une partie de la quantité d'acide totale et une partie du (des) carbonate(s) de métal alcalin et/ou alcalino-terreux de la première masse de revêtement, dans un solvant de l'acide, tel que l'eau, l'alcool ou un mélange de ceux-ci, et on les met à réagir ensemble, après quoi on applique la solution de pré-réaction ainsi préparée sur les cristaux d'acide et, après formation de la première couche de revêtement, on ajoute, en au moins une autre phase de réaction, une autre masse de revêtement pulvérisée solide et on la fait réagir, avec agitation intense, sur la couche de revêtement formée dans la phase de réaction précédente et précédemment humectée d'eau des cristaux, séparée lors de cette réaction, pour former au moins une autre couche de revêtement, après quoi, enfin, a lieu un séchage final en interrompant la formation du revêtement, caractérisé par le fait que sur les cristaux d'acide humidifiés par la solution de pré-réaction on applique un sel qui est en mesure de lier de façon irréversible, l'eau dans le complexe total et qu'on ajoute de la L-carnitine aux cristaux d'acide munis du revêtement.

2. Procédé selon la revendication 1, caractérisé par le fait qu'on applique sur les cristaux d'acide humidifiés par la solution de pré-réaction, du carbonate de sodium déshydraté, sous forme très finement pulvérisée.

3. Procédé selon la revendication 1 ou 2, caractérisé par le fait qu'on ajoute la L-carnitine en quantité telle que le granulé effervescent contienne 15 à 30% en poids de L-carnitine.

4. Procédé selon la revendication 3, caractérisé par le fait qu'on ajoute la L-carnitine en quantité telle que le granulé effervescent contienne 20 à 25% en poids de L-carnitine.

5. Granulé effervescent préparé selon l'une des revendications précédentes.

6. Utilisation du granulé effervescent selon la revendication 5 sous forme de granulé instantané pour la préparation de boissons effervescentes ou similaires.

7. Utilisation du granulé effervescent selon la revendication 5 pour la préparation de pastilles effervescentes.

## Claims

1. A method of producing an effervescent granulate containing at least one solid crystalline edible organic acid, more particularly citric acid, and at least one alkali-metal or alkaline earth metal carbonate which splits off $CO_2$ on reacting with the organic acid in an aqueous solution, in which method, in order to form a first coating layer adhering to the surface layer of the respective acid crystal via a binding layer formed by reaction between the acid and carbonate, the coating layer being made of a first pulverised coating material containing at least one alkali-metal or alkaline earth metal carbonate, a part of the total quantity of acid and a part of the alkali-metal and/or alkaline earth metal carbonate or carbonates in the first coating material are dissolved and reacted with one another in a solvent for the acid, e.g. water or alcohol or a mixture thereof, and the resulting pre-reaction solution is applied to the acid crystals and, after the first coating layer has formed, at least one additional solid pulverised coating material is added in at least one additional addition reaction step and is reacted with vigorous agitation so as to form at least one additional coating layer on the coating layer formed in the preceding addition reaction and wet from the water of crystallization split off during the addition reaction, followed finally by final drying with discontinued formation of coating, characterised in that a salt is deposited on the acid crystals wetted with the pre-reaction solution and is adapted irreversibly to bond water in the total complex, and L-carnitine is added to the coated acid crystals.

2. A method according to claim 1, characterised in that anhydrous sodium carbonate in very finely powdered form is applied to the acid crystals wetted with the pre-reaction solution.

3. A method according to claim 1 or 2, characterised in that L-carnitine is added in a quantity such that the effervescent granulate contains 15 to 30% by weight of L-carnitine.

4. A method according to claim 3, characterised in that the L-carnitine is added in a quantity such that the effervescent granulate contains 20 to 25% by weight of L-carnitine.

5. An effervescent granulate prepared according to any of the preceding claims.

6. Use of the effervescent granulate according to claim 5 as an instant granulate for producing effervescent drinks or the like.

7. Use of the effervescent granulate according to claim 5 for producing effervescent tablets.